# EUROPÄISCHE PATENTANMELDUNG

(11) **EP 3 296 303 A1**
(43) Veröffentlichungstag der Anmeldung: **21.03.2018**
(21) Anmeldenummer: 16189144.5
(22) Anmeldetag: 16.09.2016
(51) Int. Cl.: C07F 9/6574, B01J 31/18, C07B 41/06, C07C 45/50

(54) **MONOPHOSPHITE MIT METHYLENVERBRÜCKTEM DIPHENOL UND DIPHENYLPHENYLREST**

(71) Anmelder: Evonik Degussa GmbH, 45128 Essen (DE)
(72) Erfinder: DYBALLA, Katrin Marie, 45657 Recklinghausen (DE); FRANKE, Robert, 45772 Marl (DE); SELENT, Detlef, 18059 Rostock (DE); BÖRNER, Armin, 18059 Rostock (DE)

(57) **Zusammenfassung**

Monophosphite, die ein methylenverbrücktes Diphenol und einen Diphenylphenylrest aufweisen, sowie deren Verwendung als Liganden für Hydroformylierungskatalysatoren.

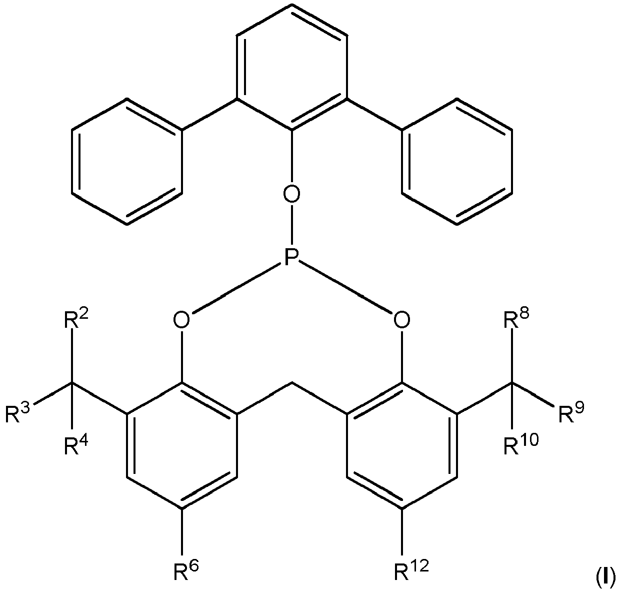

## Beschreibung

Die Erfindung betrifft Monophosphite, die ein methylenverbrücktes Diphenol und einen Diphenylphenylrest aufweisen, sowie deren Verwendung als Liganden für Hydroformylierungskatalysatoren.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor PIII. Eine gute Übersicht über den Stand der Hydroformylierung von Olefinen findet sich in B. Cornils, W. A. Herrmann, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Die Hydroformylierung führt, außer im Fall von Ethylen als Edukt, zu einer Mischung isomerer Produkte, nämlich n-Aldehyden (lineare Aldehyde) und iso-Aldehyden (verzweigte Aldehyde). Neben der Reaktionsgeschwindigkeit ist somit auch die Selektivität bei der Bildung von n- bzw. iso-Produkten ein wichtiger Parameter der Hydroformylierungsreaktion.

In WO 95/30680 (A1) werden zweizähnige Phosphinliganden und ihr Einsatz in der Katalyse, unter anderem auch in Hydroformylierungsreaktionen, offen gelegt.

Ferrocenverbrückte Bisphosphine werden beispielsweise in US 4 169 861 (A), US 4 201 714 (A) und US 4 193 943 (A) als Liganden für Hydroformylierungen beschrieben.

Der Nachteil von zwei- und mehrzähnigen Phosphinliganden ist ein relativ hoher Aufwand, der zu ihrer Darstellung notwendig ist. Daher ist es oftmals nicht rentabel, solche Systeme in technischen Prozessen einzusetzen. Hinzu kommt eine vergleichsweise geringe Aktivität, die durch hohe Verweilzeiten reaktionstechnisch kompensiert werden muss. Dies wiederum führt zu unerwünschten Nebenreaktionen der Produkte.

Die einfache Verfügbarkeit und damit verbunden die gute Möglichkeit eines großtechnischen Einsatzes ist ein wichtiges Kriterium, da der Herstellungsaufwand und damit verbunden die anfallenden Kosten nur so hoch sein dürfen, dass die Rentabilität des Gesamtprozesses weiterhin gewährleistet ist.

Rhodium-Monophosphit-Komplexe in katalytisch aktiven Zusammensetzungen sind geeignet für die Hydroformylierung von verzweigten Olefinen mit innenständigen Doppelbindungen. Seit den 1970er Jahren ist die Verwendung von so genannten "bulky phosphites" in der Hydroformylierung beschrieben (siehe u.a. van Leeuwen et. al, Journal of Catalysis, 2013, 298, 198-205). Diese zeichnen sich durch eine gute Aktivität aus, jedoch ist die n/i-Selektivität für endständig oxierte Verbindungen gering und verbesserungswürdig.

Aus EP 2 445 920 ist die Verwendung von arylsubstituierten, methylenverbrückten Diphenolen in Monophosphiten bei der rhodiumkatalysierten Hydroformylierung von Olefinen bekannt. Diese weisen jedoch sehr unterschiedliche n/i-Selektivitäten auf.

Der vorliegenden Erfindung lag vor diesem Hintergrund die Aufgabe zugrunde, neue Liganden für die Hydroformylierung bereitzustellen, welche bei vergleichbarer Ausbeute eine gesteigerte n/i-Selektivität erzielen.

Die Aufgabe wird gelöst durch eine Verbindung gemäß Anspruch 1.

Verbindung gemäß der Formel (I): 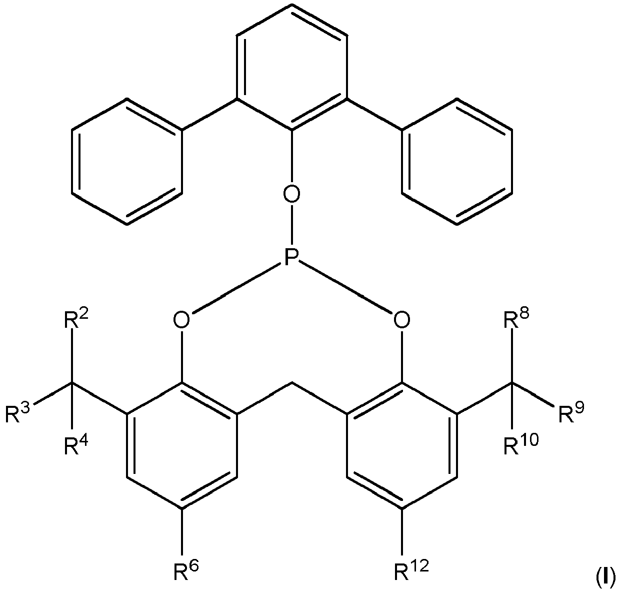
wobei R², R³, R⁴ sowie R⁸, R⁹, R¹⁰ unabhängig voneinander ausgewählt sein können aus -H, -(C₁-C₁₂)-Alkyl und Halogen, wobei jeweils zwei Substituenten aus R², R³ und R⁴ bzw. R⁸, R⁹ und R¹⁰ eine cyclische Gruppe der Formel -CH₂(CH₂)ₙCH₂- bilden können, wobei n gleich 2 oder 3 ist;
R⁶, R¹² unabhängig voneinander ausgewählt sein können aus -H, -(C₁-C₁₂)-Alkyl, O-(C₁-C₁₂)-Alkyl, -O-(C₁-C₁₂)-Alkyl-COOH, -OH und Halogen.

Die erfindungsgemäßen Phosphite gemäß Formel (I) eignen sich in besonderer Weise als Liganden für Hydroformylierungskatalysatoren. Unter Verwendung dieser Liganden lassen sich hohe Ausbeuten sowie eine sehr hohe Selektivität bei der Herstellung von n-Aldehyden aus endständigen Olefinen erzielen. Insbesondere hat sich dies bei der Hydroformylierung von n-Octen gezeigt.

Durch das Merkmal, dass jeweils zwei Substituenten aus R², R³ und R⁴ bzw. R⁸, R⁹ und R¹⁰ eine cyclische Gruppe der Formel -CH₂(CH₂)ₙCH₂- bilden können, wobei n gleich 2 oder 3 ist, soll zum Ausdruck gebracht werden, dass diese zwei Substituenten zusammen mit dem sie verbindenden Kohlenstoffatom eine Cycloalkylgruppe mit insgesamt 5 bzw. 6 Kohlenstoffatomen bilden.

Im Rahmen der Erfindung umfasst der Ausdruck -(C₁-C₁₂)-Alkyl geradkettige und verzweigte Alkylgruppen mit 1 bis 12 Kohlenstoffatomen. Vorzugsweise handelt es sich dabei um geradkettige oder verzweigte -(C₁-C₈)-Alkyl- und ganz bevorzugt um geradkettige oder verzweigte -(C₁-C₆)-Alkylgruppen. Beispiele für -(C₁-C₁₂)-Alkylgruppen sind: Methyl, Ethyl, Propyl, Isopropyl, n-Butyl, iso-Butyl, tert.-Butyl.

Die Erläuterungen zum Ausdruck -(C₁-C₁₂)-Alkyl gelten auch für die Alkylgruppen in -O-(C₁-C₁₂)-Alkyl und -O-(C₁-C₁₂)-Alkyl-COOH.

Unter Halogen werden Cl, F, Br, I zusammengefasst. Hierbei ist Cl bevorzugt.

In einer Ausführungsform können R², R³, R⁴ sowie R⁸, R⁹, R¹⁰ unabhängig voneinander ausgewählt sein aus -H, -(C₁-C₁₂)-Alkyl, wobei jeweils zwei Substituenten aus R², R³ und R⁴ bzw. R⁸, R⁹ und R¹⁰ eine cyclische Gruppe der Formel -CH₂(CH₂)ₙCH₂- bilden können, wobei n gleich 2 oder 3 ist.
Vorzugsweise ist n gleich 3.

In einer Ausführungsform können R⁶, R¹² unabhängig voneinander ausgewählt sein aus - H, -(C₁-C₁₂)-Alkyl, und -O-(C₁-C₁₂)-Alkyl.

In einer Ausführungsform stehen R⁶ und R¹² jeweils für -(C₁-C₁₂)-Alkyl.

In einer Ausführungsform stehen R⁶ und R¹² jeweils für -CH₃.

In einer Ausführungsform sind R², R³, R⁴ sowie R⁸, R⁹, R¹⁰ so gewählt, dass mindestens einer der drei Reste R², R³, R⁴ für -CH₃ steht, und mindestens einer der drei Reste R⁸, R⁹, R¹⁰ für -CH₃ steht.

In einer weiteren Ausführungsform weist die Verbindung eine der folgenden Strukturen (1) oder (**2**) auf:

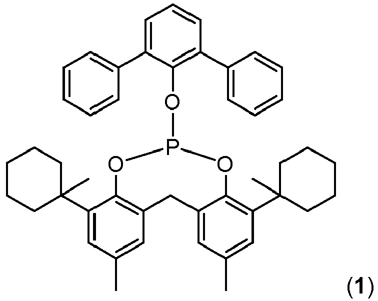

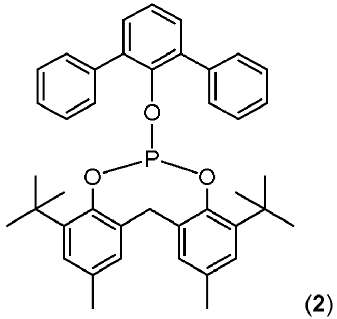

Neben den genannten Verbindungen betrifft die Erfindung auch einen Komplex, welcher die genannte Verbindung und ein Metallatom ausgewählt aus Rh, Ru, Co und Ir umfasst. In einer bevorzugten Ausführungsform ist das Metall Rh.

Die Herstellung derartiger Edelmetallkomplexe ist aus dem Stand der Technik bekannt. Siehe hierzu R. Franke, D. Selent, A. Börner, "Applied Hydroformylation", Chem. Rev., 2012, DO1:10.1021/cr3001803; S. 5688 Schema 12 "General Method forthe Preparation of a P-Modified Rh precatalyst" und darin zitierte Literaturstellen sowie P. W. N. M. van Leeuwen, in Rhodium Catalyzed Hydroformylation, P. W. N. M. van Leeuwen, C. Claver (Hrsg.), Kluwer, Dordrecht, 2000 unter anderem S. 48 ff, S.233 ff. und darin zitierte Literaturstellen sowie K.D. Wiese und D. Obst in Top. Organomet. Chem. 2006, 18, 1-13; Springer Verlag Berlin Heidelberg 2006 S. 6 ff sowie darin zitierte Literaturstellen.

Des Weiteren betrifft die Erfindung die Verwendung der Verbindung zur Katalyse einer Hydroformylierungsreaktion. Dabei wird die erfindungsgemäße Verbindung bevorzugt als Ligand in einem erfindungsgemäßen Ligand-Metall-Komplex eingesetzt. Ebenso betrifft die Erfindung die Verwendung des beschriebenen Komplexes zur Katalyse einer Hydroformylierungsreaktion. Besonders bevorzugt ist hierbei die Hydroformylierung endständiger Olefine zu Aldehyden.

Die Erfindung betrifft ebenfalls ein Verfahren, bei dem die erfindungsgemäße Verbindung als Ligand in einem Ligand-Metall-Komplex zur Umsetzung eines Olefins zu einem Aldehyd eingesetzt wird.

Das erfindungsgemäße Verfahren umfasst die Verfahrensschritte:
a) Vorlegen eines Olefins,
b) Zugabe eines erfindungsgemäßen Komplexes oder einer erfindungsgemäßen Verbindung und einer Substanz, welche ein Metallatom ausgewählt aus Rh, Ru, Co und Ir aufweist,
c) Zuführen von Wasserstoff und Kohlenstoffmonoxid,
d) Erwärmen des Reaktionsgemisches, wobei das Olefin zu einem Aldehyd umgesetzt wird.

Hierbei können die Verfahrensschritte a) bis c) in beliebiger Reihenfolge erfolgen.

In einer bevorzugten Variante des Verfahrens ist das Metallatom Rh.

Es kann hierbei auch ein Überschuss an Liganden verwendet werden und nicht zwangsläufig jeder Ligand liegt gebunden in Form eines Ligand-Metall-Komplexes vor, sondern ist als freier Ligand im Reaktionsgemisch enthalten. Vorzugsweise liegt das molare Verhältnis der erfindungsgemäßen Verbindung zum Metallatom im Bereich von 10:1 zu 1:1, bevorzugt 8:1 zu 1:1, besonders bevorzugt 6:1 zu 2:1.

Die Reaktion wird bevorzugt bei einer Temperatur von 80 °C bis 200 °C und einem Druck von 1 bar bis 300 bar durchgeführt. Besonders bevorzugt sind eine Temperatur von 100 °C bis 160 °C und ein Druck von 15 bar bis 250 bar. Besonders bevorzugt sind eine Temperatur von 110 °C bis 130 °C und ein Druck von 30 bar bis 70 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1- oder 2-Penten, 2-Methyl-1-buten, 2-Methyl-2-buten, 3-Methyl-1-buten, 1-, 2- oder 3-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Dibuten), Nonene, 2-oder 3-Methyloctene, das bei der Trimerisierung von Propen anfallende C₉-Olefingemisch (Tripropen), Decene, 2-Ethyl-1-octen, Dodecene, das bei der Tetramerisierung oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabutan) sowie durch Cooligomerisierung von Olefinen mit unterschiedlicher Anzahl von Kohlenstoffatomen (bevorzugt 2 bis 4) hergestellte Olefingemische. Bevorzugt handelt es sich um Monoolefine mit einer endständigen C-C-Doppelbindung.

### Beispiele

Die Erfindung wird anhand der folgenden Ausführungsbeispiele näher erläutert.

### Allgemeine Arbeitsvorschriften

Alle präparativen Arbeiten erfolgten unter Anwendung der Schlenk-Technik mit Argon als Schutzgas. Toluol und Tetrahydrofuran wurden mit einem Pure Solv MD-7 System gereinigt und bis zur Verwendung unter Argon aufbewahrt. Triethylamin wurde vordem Einsatz unter Argon von Natriumketyl destilliert. Phosphortrichlorid (Aldrich) wurde vor dem Einsatz unter Argon destilliert. Alle präparativen Arbeiten erfolgten in ausgeheizten Gefäßen. Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten, die halbautomatische Säulenchromatografie an einem Teledyne Isco Combiflash Rf+.

### Vorstufe 1

### 6-Chlor-2,10-dimethl-4,8-bis(1-methylcyclohexyl)-12H-dibenzo[d,g][1,3,2]dioxa-phosphocin

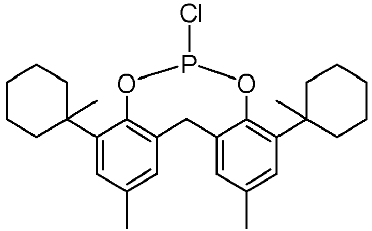

Zu einer bei 0 °C gerührten Mischung aus 6,6'-Methylen-bis(4-methyl-2-(1-methylcyclohexyl)phenol) (2,944 g; 7,0 mmol), THF (20 ml) und Pyridin (1,63 ml) wird tropfenweise eine 1,177 M Lösung von Phosphortrichlorid in THF (5,95 ml; 7,0 mmol) gegeben. Man lässt auf Raumtemperatur kommen, rührt über Nacht, filtriert und entfernt das Lösungsmittel im Vakuum. Der erhaltene weiße Feststoff wird 4 h bei 50°C/0,1 mbar getrocknet. Ausbeute: 3,331 g (6,87 mmol; 98%) mit einer spektroskopischen Reinheit von 95%.
³¹P-NMR (CD₂Cl₂): δ 156,8 (s, br) ppm.
Die Verbindung wurde wie erhalten als Edukt weiterverwendet.

### Ligand 1

### 6-([1,1':3',1"-Terphenyl]-2'-yloxy)-2,10-dimethyl-4,8-bis(1-methylcyclohexyl)-12H-dibenzo[d,g][1,3,2]dioxaphosphocin

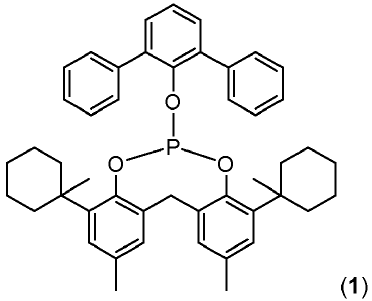

Zu einer auf 0 °C gekühlten Mischung aus 2,6-Diphenylphenol (0,385 g; 1,564 mmol), Triethylamin (2 ml) und Toluol (6 ml) wird unter Rühren eine Lösung von 6-Chlor-2,10-dimethyl-4,8-bis(1-methylcyclohexyl)-12H-dibenzo[d,g][1,3,2]dioxa-phosphocin (0,758 g; 1,564 mmol) in Toluol (4 ml) getropft. Man lässt über Nacht rühren, filtriert, entfernt das Lösungsmittel im Vakuum und trocknet den erhaltenen Feststoff bei 50°C/0,1 mbar. Umkristallisation aus heißem Heptan ergibt 0,72 g (1,036 mmol; 66%).

Elementaranalyse (ber. für C₄₇H₅₁O₃P= 694,89 g/mol): C 81,23 (81,24); H 7,45 (7,40); P 4,37 (4,46)%. ESI-TOF/HRMS: *m*/*e* 695,36495 (*M*+H)⁺.
³¹P-NMR (CD₂Cl₂): δ 136,3 (s) ppm.
¹H-NMR (CD₂Cl₂): δ 1,00 (s, 6H); 1,23-1,57 (m, 16H); 2,05 (m, 4H); 2,27 (s, 6H); 2,92 (d, *J*_{HH}= 13,0 Hz, 1H); 3,17 (d, *J*_{HH}= 13,0 Hz, 1H); 6,98 (s, br, 4H); 7,32-7,51 (m, 9H); 7,81 (m, 4H) ppm.
¹³C-NMR (CD₂Cl₂): δ 21,1; 23,2; 23,6; 26,9; 29,1; 34,0; 37,2; 38,8; 39,2; 125,5; 127,4; 128,1; 128,3; 128,5; 134,0; 136,7; 137,1; 137,2; 139,3; 140,8; 144,8 (d, *J*_{CP}= 6,7 Hz); 146,5 (d, *J*_{CP}= 10,7 Hz) ppm.

### Vorstufe 2

### 4,8-Di-tert-butyl-6-chloro-2,10-dimethyl-12H-dibenzo[d,g][1,3,2]dioxaphosphocin

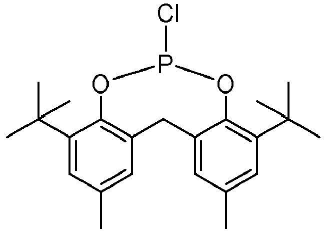

Zu einer bei 0 °C gerührten Mischung aus 6,6'-Methylen-bis(2-(*tert*-butyl)-4-methylphenol) (3,065 g; 9,0 mmol), THF (22 ml) und Pyridin (2,1 ml) wird tropfenweise eine 1,177 M Lösung von Phosphortrichlorid in THF (7,65 ml; 9,0 mmol) gegeben. Man lässt auf Raumtemperatur kommen, rührt über Nacht, filtriert und entfernt das Lösungsmittel im Vakuum. Der erhaltene weiße Feststoff wird 5 h bei 50°C/0,1 mbar getrocknet. Ausbeute: 3,5 g (8,64 mmol; 96%) mit einer spektroskopischen Reinheit von 92%.
³¹P-NMR (CD₂Cl₂): δ 154,0 (s, br) ppm. Die Verbindung wurde wie erhalten als Edukt weiterverwendet.

### Ligand 2

### 6-([1,1':3',1"-Terphenyl]-2'-yloxy)-4,8-di-tert-butyl-2,10-dimethyl-12H-dibenzo[d,g][1,3,2]dioxaphosphocin

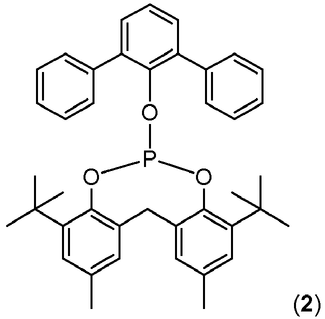

Zu einer auf 0 °C gekühlten Mischung aus 2,6-Diphenylphenol (0,520 g; 2,112 mmol), Triethylamin (2,7 ml) und Toluol (8 ml) wird unter Rühren eine Lösung von 4,8-Di-*tert*-butyl-6-chloro-2,10-dimethyl-12H-dibenzo[*d,g*][1,3,2]dioxaphosphocin (0,855 g; 2,112 mmol) in Toluol (6 ml) getropft. Man lässt über Nacht rühren, filtriert, entfernt das Lösungsmittel im Vakuum und trocknet den erhaltenen Feststoff bei 50°C/0,1 mbar. Zur Reinigung durchgeführte Umkristallisationsversuche (z. B. aus Heptan bzw. Acetonitril) waren erfolglos. Die säulenchromatografische Aufarbeitung mit Hexan/Dichlormethan (2:1, R*_{f}*= 0,5) ergab ein 85%-iges Produkt, welches wie erhalten im Katalyseversuch eingesetzt wurde.
ESI-TOF/HRMS: m/e 615,30276 (*M*+H)⁺.
³¹P-NMR (CD₂Cl₂): δ 136,3 (s) ppm.

### Durchführung der Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Das als Lösungsmittel verwendete Toluol wurde mit einem Pure Solv MD-7 System gereinigt und unter Argon aufbewahrt. Das als Substrat eingesetzte Substrat n-Octene (EVONIK Industries AG, Octenisomerengemisch aus 1-Octen: 3,3 %; *cis+trans-*2-Octen: 48,5 %; *cis*+*trans*-3-Octen*:* 29,2%; *cis*+*trans-*Octen-4: 16,4 %; gerüstisomere Octene: 2,6 %) wurde mehrere Stunden über Natrium am Rückfluß erhitzt und unter Argon destilliert.

Für die Versuche wurden im Autoklaven unter Argonatmosphäre Lösungen der Katalysatorvorstufe und des Liganden gemischt. Als Katalysatorvorstufe kam [(acac)Rh(COD)] (Umicore, acac = Acetylacetonat-Anion; COD = 1,5-Cyclooctadien) zum Einsatz. Für Versuche mit einer Konzentration von 100 ppm-m Rhodium wurden 10 ml einer 4,31 millimolaren Lösung in den Autoklaven gegeben. Anschließend wurde die einem Verhältnis L/Rh = 5:1 entsprechende Masse des Liganden in 10 ml Toluol gelöst und zugemischt. Durch Zugabe von weiterem Toluol wurde das Anfangsvolumen der Katalysatorlösung auf 41,0 ml eingestellt. In eine druckfeste Pipette wurde eingefüllt: n-Octene (10,70 g; 95,35 mmol). Der Autoklav wurde mit Synthesegas (Linde; H₂ (Qualität 5.0): CO (Qualität 4.7) = 1:1) auf einen Gesamtdruck von 42 bar gebracht und auf 120 °C aufgeheizt. Nach Erreichen der Reaktionstemperatur wurde der Synthesegasdruck im Autoklaven auf 48,5 bar erhöht und das Olefingemisch mit einem in der Druckpipette eingestellten Druck von ca. 51,5 bar in den Autoklaven gepresst. Die Reaktion wurde bei konstantem Druck von 50 bar (Nachdruckregler der Fa. Bronkhorst, NL) über 4 h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 µm.

Die Ergebnisse sind in der nachfolgenden Tabelle gezeigt.

| **Ligand** | **Ausbeute [%]** | **Selektivität [%]** |
|---|---|---|
| 1 | 97 | 31,3 |
| 2 | 94 | 30,7 |
| Alkanox 240 | 95 | 20 |

| | | |
|---|---|---|
| Selektivität % = n / (n + i) | | |

Alkanox 240 wurde unter identischen Bedingungen gemessen. Bei Alkanox 240 handelt es sich um einen aus dem Stand der Technik bekannten Triphosphitliganden der Formel:

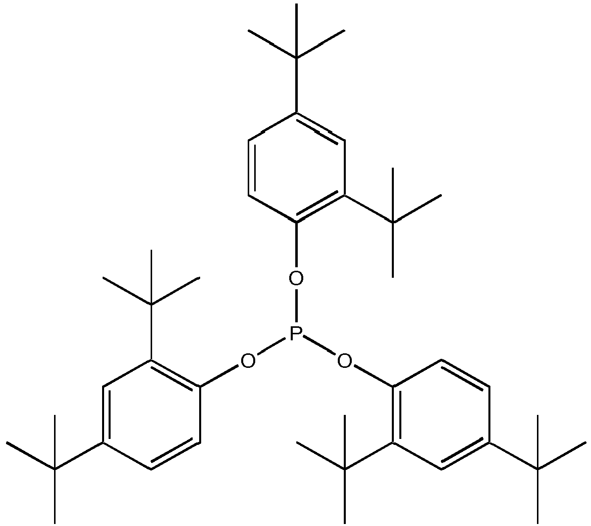

Somit konnte gezeigt werden, dass die gestellte Aufgabe durch die erfindungsgemäßen Verbindungen gelöst wird. Die Selektivität konnte gegenüber dem Vergleichsliganden (Alkanox 240) deutlich gesteigert werden.

## Patentansprüche

1. Verbindung gemäß der Formel (I): 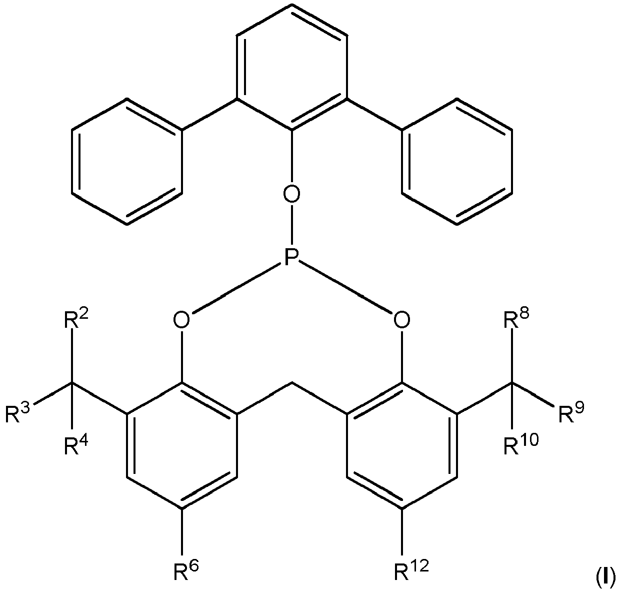
wobei R², R³, R⁴ sowie R⁸, R⁹, R¹⁰ unabhängig voneinander ausgewählt sein können aus -H, -(C₁-C₁₂)-Alkyl und Halogen, wobei jeweils zwei Substituenten aus R², R³ und R⁴ bzw. R⁸, R⁹ und R¹⁰ eine cyclische Gruppe der Formel -CH₂(CH₂)ₙCH₂- bilden können, wobei n gleich 2 oder 3 ist;
R⁶, R¹² unabhängig voneinander ausgewählt sein können aus -H, -(C₁-C₁₂)-Alkyl, O-(C₁-C₁₂)-Alkyl, -O-(C₁-C₁₂)-Alkyl-COOH, -OH und Halogen.

2. Verbindung nach Anspruch 1,
wobei R², R³, R⁴ sowie R⁸, R⁹, R¹⁰ unabhängig voneinander ausgewählt sein können aus -H, -(C₁-C₁₂)-Alkyl, wobei jeweils zwei Substituenten aus R², R³ und R⁴ bzw. R⁸, R⁹ und R¹⁰ eine cyclische Gruppe der Formel -CH₂(CH₂)ₙCH₂- bilden können, wobei n gleich 2 oder 3 ist.

3. Verbindung nach einem der Ansprüche 1 oder 2,
wobei R⁶, R¹² unabhängig voneinander ausgewählt sein können aus -H, -(C₁-C₁₂)-Alkyl, und -O-(C₁-C₁₂)-Alkyl.

4. Verbindung nach einem der Ansprüche 1 bis 3,
wobei R⁶ und R¹² jeweils für -(C₁-C₁₂)-Alkyl stehen.

5. Verbindung nach einem der Ansprüche 1 bis 4,
wobei R⁶ und R¹² jeweils für -CH₃ stehen.

6. Verbindung nach einem der Ansprüche 1 bis 5,
wobei R², R³, R⁴ sowie R⁸, R⁹, R¹⁰ so gewählt sind, dass mindestens einer der drei Reste R², R³, R⁴ für -CH₃ steht, und mindestens einer der drei Reste R⁸, R⁹, R¹⁰ für -CH₃ steht.

7. Verbindung nach einem der Ansprüche 1 bis 6,
welche eine der folgenden Strukturen (1) oder (2) aufweist:

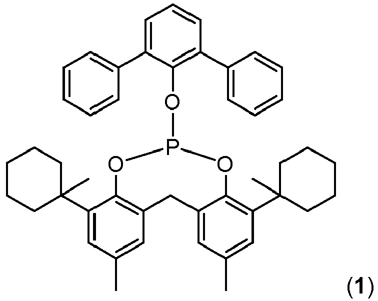

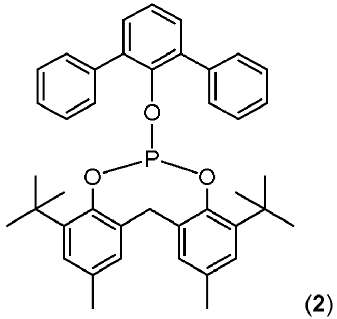

8. Komplex umfassend eine Verbindung nach einem der Ansprüche 1 bis 7 und ein Metallatom ausgewählt aus Rh, Ru, Co und Ir.

9. Verwendung einer Verbindung nach einem der Ansprüche 1 bis 7 zur Katalyse einer Hydroformylierungsreaktion.

10. Verwendung des Komplexes nach Anspruch 8 zur Katalyse einer Hydroformylierungsreaktion.

11. Verfahren zur Herstellung eines Aldehyds, umfassend die Verfahrensschritte:
a) Vorlegen eines Olefins,
b) Zugabe eines Komplexes nach Anspruch 8 oder einer Verbindung nach einem der Ansprüche 1 bis 7 und einer Substanz, welche ein Metallatom ausgewählt aus Rh, Ru, Co und Ir aufweist,
c) Zuführen von Wasserstoff und Kohlenstoffmonoxid,
d) Erwärmen des Reaktionsgemisches, wobei das Olefin zu einem Aldehyd umgesetzt wird.

## Geänderte Patentansprüche

### Geänderte Patentansprüche gemäss Regel 137(2) EPÜ.

1. Verbindung, welche eine der folgenden Strukturen (1) oder (2) aufweist:

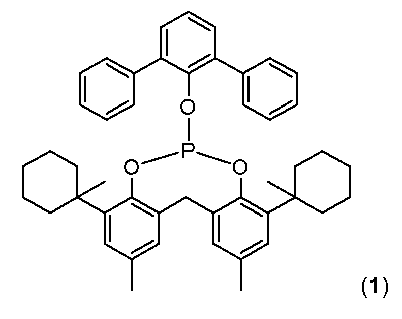

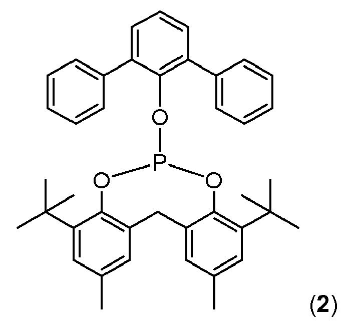

2. Komplex umfassend eine Verbindung nach Anspruch 1 und ein Metallatom ausgewählt aus Rh, Ru, Co und Ir.

3. Verwendung einer Verbindung nach Anspruch 1 zur Katalyse einer Hydroformylierungsreaktion.

4. Verwendung des Komplexes nach Anspruch 2 zur Katalyse einer Hydroformylierungsreaktion.

5. Verfahren zur Herstellung eines Aldehyds, umfassend die Verfahrensschritte:
a) Vorlegen eines Olefins,
b) Zugabe eines Komplexes nach Anspruch 2 oder einer Verbindung nach Anspruch 1 und einer Substanz, welche ein Metallatom ausgewählt aus Rh, Ru, Co und Ir aufweist,
c) Zuführen von Wasserstoff und Kohlenstoffmonoxid,
d) Erwärmen des Reaktionsgemisches, wobei das Olefin zu einem Aldehyd umgesetzt wird.
